Europäisches Patentamt

European Patent Office

Office européen des brevets

(19)

(11) Publication number: **0 490 349 B1**

## EUROPEAN PATENT SPECIFICATION

(45) Date of publication of patent specification: **31.05.95**

(51) Int. Cl.⁶: **C10G 45/64**, C07C 5/27, C07C 15/24, C10G 65/04, B01J 29/90

(21) Application number: **91121186.0**

(22) Date of filing: **10.12.91**

(54) **Process for producing 2-methylnaphthalene and method of restoring the activity of solid acid catalyst used in that process.**

(30) Priority: **12.12.90 JP 401552/90**
**28.12.90 JP 409452/90**
**12.06.91 JP 139393/91**

(43) Date of publication of application:
**17.06.92 Bulletin 92/25**

(45) Publication of the grant of the patent:
**31.05.95 Bulletin 95/22**

(84) Designated Contracting States:
**DE FR GB IT**

(56) References cited:
**EP-A- 0 351 074**
**EP-A- 0 373 740**
**DE-A- 3 723 104**
**US-A- 3 860 668**

**WORLD PATENTS INDEX LATEST, Week 3489,**
**AN 89-244097, Derwent Publications Ltd,**
**London, GB; & JP-A-1 175 946 (KAWASAKI**
**STEEL K.K.)12-07-1989**

(73) Proprietor: **KAWASAKI STEEL CORPORATION**
**No. 1-28, 1-Chome Kitahonmachi-Dori**
**Chuo-Ku, Kobe-City Hyogo 651 (JP)**

(72) Inventor: **Nobusawa, Tatsuya, c/o Technical**
**Research Division**
**Kawasaki Steel Corporation,**
**1, Kawasaki-cho**
**Chiba-shi,**
**Chiba (JP)**
Inventor: **Suzuki, Toshihide, c/o Technical Re-**
**search Division**
**Kawasaki Steel Corporation,**
**1, Kawasaki-cho**
**Chiba-shi,**
**Chiba (JP)**
Inventor: **Horita, Tsugio, c/o Technical Re-**
**search Division**
**Kawasaki Steel Corporation,**
**1, Kawasaki-cho**
**Chiba-shi,**
**Chiba (JP)**

EP 0 490 349 B1

WORLD PATENTS INDEX LAATEST, Week
3289, AN 89-232277, Derwent Publications
Ltd, London, GB; & JP-A-1 168 625
(KAWASAKI STEEL K.K.) 04-07-1989

Inventor: **Takagi, Yoshinori, c/o Technical Re-
search Division**
**Kawasaki Steel Corporation,**
**1, Kawasaki-cho**
**Chiba-shi,**
**Chiba (JP)**
Inventor: **Matsuura, Akinori, c/o Technical Re-
search Division**
**Kawasaki Steel Corporation,**
**1, Kawasaki-cho**
**Chiba-shi,**
**Chiba (JP)**

(74) Representative: **Henkel, Feiler, Hänzel & Part-
ner**
**Möhlstrasse 37**
**D-81675 München (DE)**

**Description**

Background of the Invention

Field of the Invention

This invention relates to process for producing 2-methylnaphthalene from 1-methylnaphthalene containing oils with high yield in an economical way, as well as to a method of restoring the activity of the solid acid catalyst used in that process.

Prior Art

2-Methylnaphthalene is useful as an intermediate for the synthesis of dyes and pharmaceuticals such as vitamin K. In recent years this compound has also been considered to be important as an intermediate for the synthesis of 2,6-naphthalenedicarboxylic acid which is a starting material for the manufacture of polyester resins having high heat resistance and tensile strength.

2-Methylnaphthalene is contained in the methylnaphthalene fraction which is obtained by distilling the tar resulting from dry-distillation of coals and, conventionally, basic materials are extracted from said methylnaphthalene fraction, followed by crystallization or distillation to recover 2-methylnaphthalene. The methylnaphthalene fraction that has been freed of basic materials and can be subjected to crystallization or distillation contains a large amount of 1-methylnaphthalene in addition to 2-methylnaphthalene. Hence, 1-methylnaphthalene will naturally be present in a large quantity in the residue that remains after crystallization or distillation of the methylnaphthalene fraction. In spite of its typical use as a dye, 1-methylnaphthalene has a smaller demand in industry than 2-methylnaphthalene.

The reaction for isomerization from 1-methylnaphthalene to 2-methylnaphthalene is reported by V. Solinas et al. in Applied Catalysis, 9, 1984, pp. 109 - 117 and ibid., 5, 1983, pp. 171 - 177 in connection with various types of zeolite that were used as isomerization catalysts. In their papers, Solinas et al. described the activity of those catalysts, their durability and the method of regenerating them.

Japanese Patent Publication No. 21018/1980 (corresponding to U.S. Patent No. 3,860,668) describes isomerization of an alkylbenzene, in particular, mixed xylene, in the presence of a silica alumina catalyst. According to this patent, a cyclic hydrocarbon as exemplified by tetralin, decalin or cyclohexane is additionally used to improve the selectivity for paraxylene in the isomerization of the mixed xylene and to reduce the carbon deposition on the catalyst.

DE-A-37 23 104 discloses a process for selectively producing 2-methylnaphthalene by isomerizing 1-methylnaphthalene in the presence of a zeolite or a zeolite-like solid having a spaciousness index in the range of 2 to 19.

JP-A-1 168 625 discloses a process for producing purified naphthalene
(1) by applying high pressures to hydrogenated naphthalene obtained by hydrogenation of 95% naphthalene or to oily by-product obtained after compressing the hydrogenated naphthalene in manufacturing purified naphthalene and
(2) solid liquid separation of the resultant crystals from mother liquor at 70 to 90°C and 150 to 1000 kg/cm$^2$.

JP-A-1 175 946 discloses a process for producing 2-methylnaphthalene by isomerizing 1-methylnaphthalene in the presence of a solid super strong acid as a catalyst at temperatures of 50 to 400°C.

Summary of the Invention

The present inventors conducted intensive studies on a process for producing 2-methylnaphthalene which comprises subjecting 1-methylnaphthalene containing feed oil to an isomerization reaction in the presence of a solid acid catalyst so as to obtain a composition containing 1-methylnaphthalene and 2-methylnaphthalene at equilibrium and then recovering 2-methylnaphthalene from said equilibrium composition. As a result, the present inventors learned that this method suffered from the problem that the activity of the solid acid catalyst used in the isomerization reaction would drop within a very short period of time. The present inventors also learned that solving this problem was very important for the purpose of establishing a process for economical and advantageous production of 2-methylnaphthalene from a 1-methylnaphthalene containing feed oil.

Under these circumstances, the general object of the present invention is to provide a process by which 2-methylnaphthalene can be produced from a 1-methylnaphthalene containing oil in an economically advantageous way.

A first specific object of the present invention is to provide a process in which the activity of a catalyst for isomerizing 1-methylnaphthalene in a 1-methylnaphthalene containing oil to 2-methylnaphthalene is retained for an extended period so that 2-methylnaphthalene can be obtained from the feed oil economically with high yield.

A second specific object of the present invention is to provide a method by which a catalyst the activity of which has been deteriorated as a result of its use in isomerization of 1-methylnaphthalene in a 1-methylnaphthalene containing oil to 2-methylnaphthalene can be regenerated efficiently without interrupting the operation of the isomerization step.

The present inventors conducted intensive studies in order to attain those objects and found as a result that the activity of the solid acid catalyst used for isomerization of 1-methylnaphthalene to 2-methylnaphthalene could be sustained for a remarkably extended period with a high selectivity for 2-methylnaphthalene by performing the isomerization in the presence of tetralin or an alkyltetralin. Japanese Patent Publication No. 21018/1980, supra (corresponding to USP 3,860,668) is concerned primarily with the isomerization of mixed xylene consisting of p-xylene, m-xylene, o-xylene and ethylbenzene and it has nothing to do with isomerization from 1-methylnaphthalene to 2-methylnaphthalene. Furthermore, this patent has no reference at all to the relationship between the life of the catalyst activity and the presence of tetralin or other cyclic hydrocarbons as they are used in the isomerization of mixed xylene. Therefore, it was entirely unexpected that the activity of the catalyst for isomerization of 1-methylnaphthalene in a 1-methylnaphthalene containing oil to 2-methylnaphthalene could be retained for a prolonged time with a high yield for 2-methylnaphthalene by performing that isomerization in the presence of tetralin or alkyltetralins.

In the course of their continued studies, the present inventors performed an isomerization reaction on a 1-methylnaphthalene containing oil after hydrodesulfurization and found unexpectedly that the activity of the solid acid catalyst for the isomerization reaction could be prolonged with a high yield for 2-methylnaphthalene. In order to identify the reason for this surprising fact, the present inventors conducted further investigation and found the following facts: 1) when the 1-methylnaphthalene containing oil was subjected to hydrodesulfurization, part of the methylnaphthalene in the oil underwent nuclear hydrogenation to produce a small amount of methyltetralin, which effectively prevented the deactivation (deterioration) of the solid acid catalyst so that its activity could be retained for a prolonged period with a high yield for 2-methylnaphthalene; and 2) by using the thus produced methyltetralin so as to control appropriately the amount of methyltetralin to be supplied to the subsequent isomerization step, the activity of the solid acid catalyst used in the isomerization step could be extended for a longer period.

Hydrodesulfurization is generally conducted to remove sulfur compounds and it was quite unexpected and surprising that the isomerization of 1-methylnaphthalene to 2-methylnaphthalene could be accomplished efficiently over a prolonged period with a high yield for 2-methylnaphthalene when it was preceded by the step of hydrodesulfurization. Needless to say, by performing hydrodesulfurization, any unwanted sulfur compounds can be removed and the 2-methylnaphthalene obtained by subsequent isomerization has a such a high purity.

The present inventors also found that the activity of she solid acid catalyst whose activity dropped as a result of its use in the reaction of isomerization of 1-methylnaphthalene in a 1-methylnaphthalene containing oil to 2-methylnaphthalene could be restored effectively by adding tetralin or alkyltetralins to the isomerization reaction zone.

The present invention has been accomplished on the basis of those discoveries by the present inventors and the aforementioned objects can be attained by this invention.

The present invention generally encompasses the following six embodiments.

In its first embodiment, the present invention provides a process producing 2-methylnaphthalene which comprises performing an isomerization reaction on a 1-methylnaphthalene containing feed oil by contacting said feed oil with a solid acid catalyst in the presence of at least one compound selected from the group consisting of tetralin and alkyltetralins, and recovering 2-methylnaphthalene from the resulting reaction product.

In its second embodiment, the present invention provides a process for producing 2-methylnaphthalene according to the first embodiment, wherein 1-methylnaphthalene containing oil is subjected to a hydrodesulfurization step prior to the isomerization reaction step and part or all of the by-product methyltetralin of the hydrodesulfurization step is supplied to the isomerization step.

In its third embodiment, the present invention provides a process for producing 2-methylnaphthalene according to the first and second embodiment, wherein the 1-methylnaphthalene containing feed oil is a hydrodesulfurized, methyltetralin containing 1-methylnaphthaline containing feed oil.

4

In its fourth embodiment, the present invention provides a process for producing 2-methylnaphthalene according to the first embodiment, wherein 1-methylnaphthalene containing oil is subjected to a hydrogenation step prior to the isomerization reaction step and part or all of the by-product methyltetralin of the hydrogenation step is supplied to the isomerization step.

In its fifth embodiment, the present invention provides a process for producing 2-methylnaphthalene according to the first and second embodiment, wherein the 1-methylnaphthalene containing feed oil is a hydrogenated, methyltetralin containing 1-methylnaphthalene containing feed oil.

In accordance with these five embodiments, the aforementioned first specific objects of the present invention can be attained.

In its last and sixth embodiment, the present invention provides a method of restoring the activity of a solid acid catalyst for isomerization of 1-methylnaphthalene to 2-methylnaphthalene, in which a solid acid catalyst whose activity has deteriorated as a result of its use in the reaction of isomerization of a 1-methylnaphthalene containing feed oil is brought into contact under reaction conditions for isomerization with at least one compound selected from the group consisting of tetralin and alkyltetralins.

In accordance with this last embodiment, the aforementioned second specific object of the present invention can be attained.

Brief Description of the Drawings

The objects, features and merits of the present invention will be understood in a better way by referring to the accompanying drawings, in which:

Fig. 1 is a graph showing the experimental results of Example 1 and Comparative Example 1 in terms of the time-dependent change in the conversion of 1-methylnaphthalene in the isomerization reaction;

Fig. 2 is a graph showing the experimental results of Example 2 and Comparative Example 2 in terms of the time-dependent change in the conversion of 1-methylnaphthalene in the isomerization reaction;

Fig. 3 is a graph showing the experimental results of Example 3 and Comparative Example 3 in terms of the time-dependent change in the conversion of 1-methylnaphthalene in the isomerization reaction;

Fig. 4 is a flowchart for a specific implementation of the second embodiment of the present invention;

Fig. 5 is a flowchart for a specific implementation of the third embodiment of the present invention, and

Fig. 6 is a graph showing the experimental results of Example 11 and Comparative Example 5 in terms of the time-dependent change in the conversion of 1-methylnaphthalene in the isomerization reaction.

Detailed Description of the Invention

The more preferred embodiments of the present invention and their merits will become apparent as it is described below in detail.

First embodiment of the invention

To begin with, the first embodiment of the present invention is described.

Example of the 1-methylnaphthalene containing oil include pure 1-methylnaphthalene and an oil that is a mixture of 1-methylnaphthalene and 2-methylnaphthalene and that contains 2-methylnaphthalene at a concentration lower than that of thermodynamically equilibrium composition of 1-methylnaphthalene and 2-methylnaphthalene. In case of a feed oil that contains 2-methylnaphthalene at concentrations higher than that of thermodynamically equilibrium composition of 1-methylnaphthalene and 2-methylnaphthalene, it is advantageous for the purpose of 2-methylnaphthalene production to separate the already present 2-methylnaphthalene, with the remainder being used as a feed to be isomerized. Other hydrocarbons such as naphthalene may be present as impurities in the feed oil. Specific examples of the 1-methylnaphthalene containing oil include coal tar and petroleum fractions that contain 1-methylnaphthalene. More specific examples include a methylnaphthalene fraction which contains 1-methylnaphthalene and 2-methylnaphthalene, from which basic materials have been removed, as well as the liquid residue that remains after 2-methylnaphthalene is recovered by crystallization or distillation from said methylnaphthalene fraction. Content of nitrogen containing compounds as impurities in the 1-methylnaphthalene containing feed oil is preferably 0.2 wt% or less, more preferably, 0.1 wt% or less, as calculated for the nitrogen atom.

Generally, it is preferred for industrial purposes that the feed oil contains 1-methylnaphthalene in an amount of at least 10 wt%, with the amount of at least 20 wt% being particularly preferred.

In the first embodiment of the present invention, the isomerization reaction is performed in the presence of at least one compound selected from the group consisting of tetralin and alkyltetralins. Alkyltetralins are tetralin compounds having 1 - 8 alkyl group of 1 - 4 carbon atoms, which are bonded to a tetralin ring on at least one position of 1 - 8. Exemplary alkyl groups include methyl, ethyl, propyl and isopropyl. These compounds may be used either on their own or as admixtures. Of these compounds, methyltetralin is the most preferred because it is dehydrogenated during the isomerization reaction to be converted to methylnaphthalene, which is further isomerized to 2-methylnaphthalene, eventually leading to a higher yield of 2-methylnaphthalene.

If the isomerization reaction is performed in the presence of such tetralin or alkyltetralins (the two groups of compounds are hereinafter collectively referred to as "tetralins"), the activity of the solid acid catalyst is prolonged remarkably but the exact reason for this phenomenon has not yet been fully unravelled.

Tetralins may be mixed with the feed oil before it is supplied to the reactor or, alternatively, they may be fed to the reactor separately from the feed oil. Tetralins may be used at any proportions with respect to the feed oil but, preferably, tetralins account for 0.1 - 90% of the total weight including the feed oil, with the range of 0.2 - 50% being more preferred. The particularly preferred range is 0.4 - 10%. If the proportion of tetralins is too small, they are not completely effective in prolonging the activity of the solid acid catalyst and in obtaining the high selectivity for isomerization of 1-methylnaphthalene to 2-methylnaphthalene. If the proportion of tetralins is too great, the materials that do not participate directly in the isomerization reaction will be fed to the reactor for isomerization, as well as to the subsequent step of purification for recovering 2-methylnaphthalene, and this is not only inefficient but also uneconomical.

Any solid acid catalyst may be used in isomerization as long as it has the activity for isomerizing 1-methylnaphthalene to 2-methylnaphthalene. Specific example of useful solid acid catalysts include silica-alumina, alumina and zeolite. Among them, Y zeolite is preferred and dealuminated Y zeolite is particularly preferred. Dealuminated Y zeolite is superior to other types of zeolite in that its catalytic activity can be sustained for a longer time and that, therefore, the addition of tetralins can accordingly be reduced. Dealumination can be accomplished by a steam or acid treatment, which methods are known per se.

The solid acid catalysts have the ability to dehydrogenate tetralins. Therefore, if methyltetralin is used, methylnaphthalene is produced by the dehydrogenation reaction and the subsequent isomerization reaction will produce 2-methylnaphthalene, so that not only can the yield of the product 2-methylnaphthalene be increased but also decrease of its purity due to the addition of tetralins can be minimized.

The isomerization reaction may be performed by either a continuous flow system or a batch system but for the purpose of mass-production on an industrial scale, an continuous flow system is advantageous from an economic viewpoint. The reaction may be carried out in either a vapor or a liquid phase. The reaction temperature is normally in the range of 300 - 600 °C and reaction pressure is either atmospheric or superatmospheric; in the latter case, the pressure may be close to one atmosphere or much higher than that. If desired, the isomerization reaction may be performed in a vapor phase at low pressures of ca. 0 - 10 kg/cm$^2$G or in a liquid phase at high pressures of ca. 10 - 50 kg/cm$^2$G.

When performing the isomerization reaction in a vapor phase, one may or may not use a diluent gas such as nitrogen, water vapor, or hydrogen. For longer maintenance of the catalytic activity, hydrogen is preferably used as a diluent gas. The higher the degree of dilution, the longer the period for which the activity of the solid acid catalyst can be sustained and the higher selectivity for 2-methylnaphthalene can be obtained. However, if tetralins are allowed to be present in adequate quantities, the catalytic activity can be maintained for a satisfactorily long period even if only a small amount of diluent gas is used. If tetralins are present in amounts greater than a certain level that depends on the kind and amount of catalyst use, there is no substantial loss in the catalytic activity even in the absence of a diluent gas. Thus, saving on the diluent gas is possible according to the first embodiment of the present invention.

In the isomerization reaction, WHSV (the weight of 1-methylnaphthalene passing over a unit weight of catalyst per hour) is preferably in the range of 0.1 - 20 h-1. If WHSV is too large, a satisfactorily high conversion of 1-methylnaphthalene cannot be attained. If WHSV is too small, the catalyst packing and the reactor's capacity will increase to cause diseconomy.

By the procedure described above, 1-methylnaphthalene in the 1-methylnaphthalene containing oil is isomerized to 2-methylnaphthalene, which is recovered from the resulting reaction product by a suitable technique such as distillation.

The first embodiment of the present invention offers the following merits:

(1) Because of the presence of tetralins, the life of the solid acid catalyst used in the isomerization step is extended remarkably with a high yield for 2-methylnaphthalene, thereby making it possible to recover 2-methylnaphthalene from the 1-methylnaphthalene containing oil in an economically advantageous manner;

(2) If methyltetralin is used as a specific example of tetralins in the isomerization step, it is converted to 2-methylnaphthalene as a result of dehydrogenation and isomerization reactions that occur in the isomerization step and this improves not only the overall yield of 2-methylnaphthalene but also decrease of its purity due to the addition of tetralins can be minimized; and

(3) Saving on diluent gases can be accomplished.

Second and third embodiments

The second and third embodiments of the present invention are described below.

The 1-methylnaphthalene containing feed oil to be used as the starting material in those embodiments may be substantially the same as the feed oil used in the above-discussed first embodiment. A preferred feed oil for use in these embodiments is a methylnaphthalene containing oil that is free from tar basic compounds and indole and that contains at least 80 wt%, especially at least 90 wt%, of methylnaphthalenes (i.e., 1-methylnaphthalene and 2-methylnaphthalene) before hydrodesulfurization.

Such methylnaphthalene-containing oils can be readily prepared by known methods from treated oils which are generally obtained by fractional distillation of coal tar. For example, tar basic compounds may be removed by scrubbing with sulfuric acid or azeotropic distillation; indole may be removed by solvent extraction, alkali fusion and/or oligomer chlorination, azeotropic distillation, etc; and hydrocarbon compounds other than methylnaphthalene may be separated and removed by distillation.

If the total content of 1-methylnaphthalene and 2-methylnaphthalene before hydrodesulfurization is less than 80 wt%, the energy load for unit operations such as isomerization and 2-methylnaphthalene recovery will increase, so the total content of two methylnaphthalene isomers is preferably at least 80 wt%, more preferably at least 90 wt%. Above 80 wt%, methyltetralin can be obtained by hydrodesulfurization in the necessary and sufficient amount for the subsequent isomerization step.

The solid acid catalyst and the other conditions for the isomerization reaction to be performed in the second and third embodiments of the present invention may be identical to those described in connection with the first embodiment.

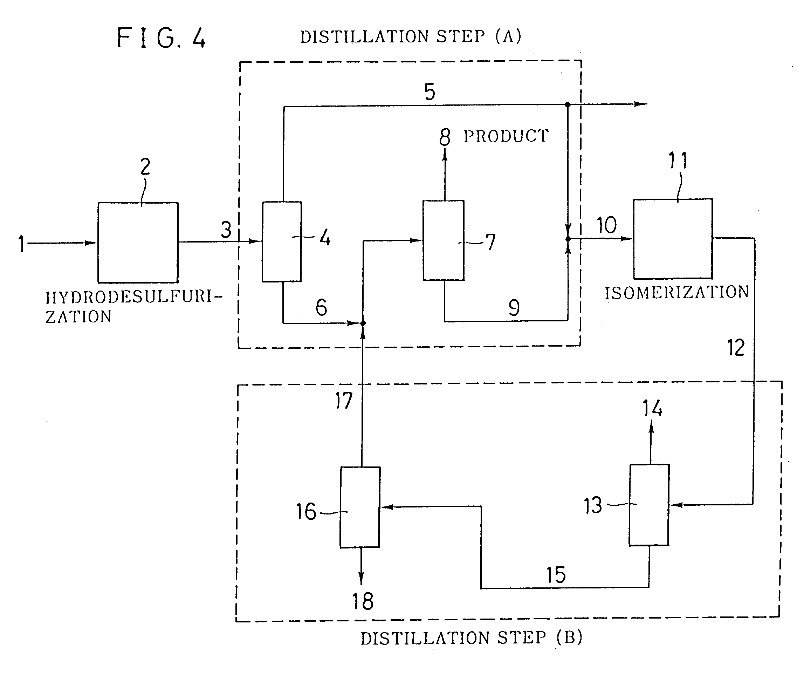

An example of the process for producing 2-methylnaphthalene in accordance with the second embodiment is described below with reference to the flowsheet shown in Fig. 4. It should, however, be noted that the second embodiment is in no way limited to that particular flowsheet and the intended object can be attained by changing the combination of individual steps in appropriate ways.

The process starts with supplying a methylnaphthalene-containing feed oil 1 into a hydrodesulfurization vessel 2 to perform hydrodesulfurization, which is the first step of the process for 2-methylnaphthalene production in accordance with the present invention. One of the function of the hydrodesulfurization step is to remove any sulfur compounds from the methylnaphthalene containing oil and it can be accomplished by any known methods of hydrodesulfurization. The percent removal of sulfur compounds may be determined in accordance with the specifications of the final product and is not limited to any particular value. As a side reaction, the nuclear hydrogenation of methylnaphthalene occurs to produce methyltetralin but there is no particular need to monitor the amount of its generation. This is because of the following two reasons: if the total concentration of 1-methylnaphthalene and 2-methylnaphthalene in the methylnaphthalene-containing oil to be hydrodesulfurized is at least 80 wt%, methyltetralin will be generated by hydrodesulfurization in a sufficient amount for the subsequent isomerization step; and by providing a distillation step as a preliminary step for isomerization following hydrodesulfurization, the concentration of methyltetralin in the methylnaphthalene containing oil to be supplied to the isomerization step can be controlled. Further, if the 1-methylnaphthalene containing oil involves a small amount of naphthalene, part of it will be converted to tetralin in the hydrodesulfurization step, which tetralin serves to prevent the decrease in the activity of the solid acid catalyst during the subsequent isomerization step.

The catalyst to be used in hydrodesulfurization is such that both molybdenum and at least one metal selected from the group consisting of cobalt and nickel are carried on an alumina support, as exemplified by cobalt/molybdenum on alumina, nickel/molybdenum on alumina, and cobalt/nickel/molybdenum on alumina. These catalysts are commercially available on the market. These are not the sole examples of the catalyst that can be used in the hydrodesulfurization step and any other catalysts may be used as long as they are capable of achieving both hydrodesulfurization and an appropriate but by no means excessive degree of nuclear hydrogenation of methylnaphthalene. In addition, elements other than molybdenum, cobalt and nickel may be present in amounts that are by no means detrimental to the purposes of the present invention.

The temperature for the hydrodesulfurization reaction ranges from 240 to 400 °C, preferably from 260 to 350 °C, and the reaction pressure ranges from atmospheric to 100 kg/cm$^2$G, preferably from 0 - 20

$kg/cm^2G$. If the temperature and pressure are set to values lower than the ranges specified above, the reaction rate for hydrodesulfurization is too low to achieve the intended degree of hydrodesulfurization. If the temperature and pressure settings are higher than the ranges specified above, hydrogenation of methylnaphthalene will become so extensive that the yield of 2-methylnaphthalene will decrease. Generally speaking, the degree of hydrogenation of methylnaphthalene tends to increase as the degree of desulfurization increases. The liquid space velocity LHSV (the amount of the feed oil as supplied per liter of catalyst) in the hydrodesulfurization step usually ranges from 0.2 to 10.0 L/L•hr. The ratio of hydrogen flow (GHSV in $hr^{-1}$) to liquid space velocity (LHSV in $hr^{-1}$) in the hydrodesulfurization step is preferably at least 30. If GHSV/LHSV is less than 30, the catalyst activity for hydrodesulfurization will drop markedly.

By treating the 1-methylnaphthalene containing feed oil under the conditions described above, the sulfureous compounds present in the feed oil are hydrocracked to light boiling materials, which are removed by a suitable means such as distillation, whereby the sulfureous compounds are eliminated from the 1-methylnaphthalene containing oil.

Following the hydrodesulfurization step, 2-methylnaphthalene is separated as the product in the next distillation step (A). The distillation step (A) consists of a distillation column 4 and a rectifying column 7. In the distillation column 4, impurities having lower boiling points than 2-methylnaphthalene (said impurities being primarily composed of methyltetralin, occasionally containing tetralin and naphthalene) are separated as a top fraction 5 from the hydrodesulfurized oil 3, with a fraction 6 being obtained from the bottom of the column 4. The bottom fraction 6 being obtained from the bottom of the column 4. The bottom fraction 6 combines with the top fraction 17 coming from distillation column 16 in the distillation step (B) and the combined flow is supplied to the rectifying column 7, where the product 2-methylnaphthalene is separated as a fraction 8, leaving a fraction 9 in the column 7. The conditions for operations in the distillation column 4 and the rectifying column 7 may be set in accordance with the specifications of the final product and in such a way that a feed 10 to the subsequent step of isomerization (which is the combined flow of fractions 5 and 9) will have a 1-methylnaphthalene content of at least 35 wt%, preferably at least 50 wt%. If the fraction 10 which is the feed to the isomerization step has a 1-methylnaphthalene content of less than 35 wt%, the efficiency of the isomerization process will decrease from the thermodynamic equilibrium viewpoint of the isomerization reaction. There is no particular upper limit of the 1-methylnaphthalene content in the fraction 10 if said content is not lower than 35 wt% and a suitable value may be determined in consideration of the design and operational critical values for the distillation column 4 and 7.

The fraction 10 from the distillation step (A) is supplied to an isomerization vessel 11 as the feed to the isomerization step, where part of 1-methylnaphthalene is isomerized to 2-methylnaphthalene. In the isomerizing step, solid acid catalysts such as zeolite and silica/alumina are used. A preferred solid acid catalyst is zeolite, with Y zeolite being more preferred. As particularly preferred solid acid catalyst is dealuminated Y zeolite which has a longer life than the silica/alumina or non-dealuminated Y zeolite catalyst.

In the isomerization step, WHSV (the weight of the feed oil passing over a unit weight of catalyst per hour) is preferably in the range of 0.1 - 20 $h^{-1}$. If WHSV is too large, a satisfactorily high conversion of 1-methylnaphthalene cannot be attained. If WHSV is too small, the catalyst packing and the reactor's capacity will increase to cause diseconomy. The reaction temperature for the isomerization step ranges from 300 to 600 °C, preferably from 350 to 550 °C. The reaction pressure is either atmospheric or superatmospheric; in the latter case, the pressure may be close to one atmosphere or much higher than that. If desired the isomerization reaction may be performed in a vapor phase at low pressure of 0 - 10 $kg/cm^2G$ or in a liquid phase at high pressure of 10 - 50 $kg/cm^2G$.

The fraction 5 from the top of the distillation column 4 containing methyltetralin, occasionally further containing tetralin, and part or all of this fraction is supplied to the isomerization step. In this way, the activity of the solid acid catalyst to be used in the isomerization step can be prolonged markedly.

The content of methyltetralin in the fraction 10 or the total content of methyltetralin and tetralin (if it also contains tetralin) is preferably in the range of 0.1 - 20.0 wt%, with the range of 0.2 - 10.0 wt% being more preferred. The most preferred range is 0.4 - 8.0 wt%. If the contents of those hydrogenation products are too small, they are not highly effective in preventing the loss of catalytic activity. If their contents are excessive, disproportionation and other side reactions that are distinct from the intended isomerization reaction will take place so extensively that the selectivity for 2-methylnaphthalene decreases.

The content of methyltetralin or the total content of methyltetralin and tetralin in the fraction 10 can be adjusted by controlling the flow rate of fraction 5 to be combined with the fraction 9.

A fraction 12 emerging from the isomerization step is subsequently sent to the distillation step (B), which is composed of distillation columns 13 and 16. The function of the distillation step (B) is to insure that the low-boiling and high-boiling substances generated in the isomerization and hydrodesulfurization steps are distilled off from the system. Stated more specifically, a fraction 14 having a lower boiling point than methylnaphthalene is distilled off in column 13 to go outside of the system, whereas a high-boiling fraction 18 is distilled off in column 16 to outside of the system. The top fraction 17 from the column 16 is circulated to the distillation step (A) where it combines with the fraction 6.

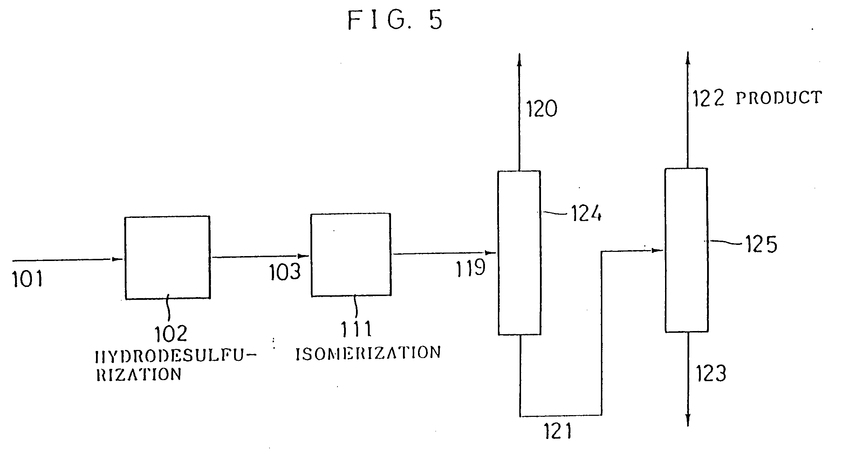

The third embodiment of the present invention is next described below with reference to the flow sheet shown in Fig. 5. In this embodiment, a 1-methylnaphthalene containing oil 101 is first subjected to a hydrodesulfurization step 102 and the resulting hydrodesulfurized oil 103 is supplied to an isomerization step 111, with 2-methylnaphthalene being recovered as the final product from the reaction product 119. In the flow sheet shown in Fig. 5, distillation is adopted as a means of recovering 2-methylnaphthalene; a light boiling fraction 120 is removed in a distillation column 124 and a high-boiling fraction 123 is removed in a distillation column 125, with 2-methylnaphthalene fraction 122 being obtained as the product.

The operating procedure and conditions for the hydrodesulfurization step to be performed in the third embodiment may be identical to those which are already described in connection with the second embodiment and this is also the case for the isomerization step.

Referring to Fig. 5, the low-boiling fraction 120 contains methyltetralin, occasionally further containing tetralin, and part of said fraction may be circulated to the isomerization step.

The above-described second and third embodiments of the present invention will offer the following merits:

(1) A small amount of methyltetralin, which is a by-product that is generated in the step of hydrodesulfurization of the 1-methylnaphthalene containing oil prior to isomerization, helps to extend the life of the solid acid catalyst used in the subsequent isomerization step;

(2) The isomerizing solid acid catalyst has the ability to dehydrogenate methyltetralin, so the methyltetralin produced in the hydrodesulfurization step is dehydrogenated to methylnaphthalene, the 1-methylnaphthalene content of which is isomerized to 2-methylnaphthalene, thereby contributing to a higher yield and purity of the product 2-methylnaphthalene; and

(3) By combining the steps of hydrodesulfurization, isomerization and distillation in an appropriate manner, the percent conversion of 1-methylnaphthalene to 2-methylnaphthalene can be increased to a very high level, whereby 2-methylnaphthalene can be obtained in a high overall yield.

Forth and fifth embodiments

These embodiments of the present invention are preferably applicable to the case where the 1-methylnaphthalene containing feed oil is of such a low sulfur content that there is no particular need to remove sulfur containing compounds by hydrodesulfurization. The preferred composition except sulfur of the feed oil may be the same as those described in connection with the second embodiment of the present invention.

Flowsheets for the production process according to the fourth and fifth embodiments may be constructed as already described in connection with the second and third embodiments, respectively, except that a hydrogenation step is adopted in place of the hydrodesulfurization step.

Hydrogenation may be performed under the same conditions and by the same procedure as in the already-described hydrodesulfurization step. Hydrogenation is not intended to achieve desulfurization but it needs only to produce methyltetralin by causing nuclear hydrogenation of methylnaphthalene, so the conditions of hydrogenation may be milder than those employed in the hydrodesulfurization step. Speaking of a hydrogenation catalyst, it may be a metal oxide containing at least one metal selected from among Ni, Cu and Cr, a metal-metal oxide catalyst such as copper - chromium oxdie, a stabilized nickel catalyst, or a noble metal catalyst containing either Pt or Pd or both. As other type of catalysts for the hydrogenation, nickel catalyst carried on a diatomaceous or a silicon dioxide, Raney nickel catalyst, noble metal catalyst carried on a carbon including Pd, Pt or Rh are exemplified. The typical pressure and temperature conditions for hydrogenation are within the ranges of from 0 to 100 $kg/cm^2G$ and from 100 to 500 °C.

In any event, it is recommended that as in the second and third embodiments, the hydrogenation step be performed in appropriate combinations with the distillation and isomerization steps so that the content of methyltetralin in the feed oil to the isomerization step and, if it also contains tetralin, the total content of methyltetralin and tetralin is preferably in the range of 0.1 - 20.0 wt%, more preferably 0.2 - 10.0 wt%.

The fourth and fifth embodiments of the present invention offer the same merits as the second and third embodiments.

Sixth embodiment

This embodiment of the present invention concerns a method by which the solid acid catalyst the activity of which has deteriorated as a result of its use in the reaction for isomerization of a 1-methylnaphthalene containing oil can be regenerated in an efficient way. In this embodiment, the 1-methylnaphthalene containing oil, the solid acid catalyst to be used in the isomerization reaction and the conditions for isomerization reaction are entirely the same as already described in connection with the first embodiment, except that the isomerization reaction is carried out without addition of tetralins.

If the isomerization reaction is performed in the manner described above, the activity of the solid acid catalyst will drop sharply. In order to restore its activity, the deteriorated solid acid catalyst is regenerated by contacting it with tetralins, whereupon the activity of the catalyst is restored. This regenerating treatment can be performed with or without interruption of the supply of the 1-methylnaphthalene containing feed oil to the reaction vessel for isomerization (isomerizer). If the supply of the feed oil is not interrupted, the regeneration of the catalyst can be performed by adding tetralins to the feed oil.

The regenerating treatment may be performed in either a vapor or liquid phase. When performing regeneration in a vapor phase with tetralins being added to the feed oil, the temperature is preferably in the range of 300 - 600°C, the pressure 0 - 10 kg/cm$^2$G, WHSV 0.1 - 20 hr$^{-1}$, and the addition of tetralins preferably accounts for at least 0.01 wt% of the sum of the feed oil and tetralins. A diluent gas may or may not be used and if it is used at all, hydrogen gas is preferred and the volume ratio of the diluent gas to the gasified feed oil is preferably not higher than 100.

If this embodiment is adopted, the reaction of isomerization is permitted to proceed while, at the same time, the activity of the deteriorated catalyst can be effectively restored.

If the processing temperature is too low, the addition of tetralins will not prove satisfactorily effective. If the processing temperature is too high, the deterioration of the catalyst will be accelerated rather than retarded. The higher the pressure, the higher the reaction rate of isomerization. However, at excessive pressures, the energy cost increases and an economic disadvantage will occur. If the WHSV is too low, not only does diseconomy result from the need to increase the capacity of the reactor but at the same time a side reaction will proceed to yield a great amount of by-products. If the WHSV is too high, a satisfactory conversion of 1-methylnaphthalene cannot be attained. If the content of tetralins in the feed oil to be supplied to the reactor is too low, the intended effect of catalyst regeneration cannot be achieved. If the content of tetralins in the feed oil is too high, undesired products will flow out of the reactor in large amounts to cause an economic disadvantage. The diluent gas to accompany the feed oil is most preferably hydrogen. If the ratio of hydrogen to the feed oil is too high, the concentration of tetralins in the vapor phase becomes too low to achieve their intended effect. Furthermore, the hydrogen cost increases to an economically disadvantageous level.

Methyltetralin may be used as a specific example of the tetralins that are to be brought into contact with the solid acid catalyst to be regenerated in the manner described above. In this case, methyltetralin is dehydrogenated and isomerized to 2-methylnaphthalene. Hence, it is effectively used for not only increasing the yield of 2-methylnaphthalene but also for maintaining the high purity of the final product.

The sixth embodiment of the present invention which has been described above in detail offers the following merit:

(1) The activity of the solid acid catalyst that has been deteriorated as a result of its use in the reaction for isomerization of a 1-methylnaphthalene containing oil can be restored within a short period by a simple procedure without interrupting the reaction and, hence, 2-methylnaphthalene can be obtained from the 1-methylnaphthalene containing oil in an economically advantageous manner.

The following examples are provided for the purpose of further illustrating the present invention. In the following description, 1-methylnaphthalene and 2-methylnaphthalene are sometimes abbreviated as 1-MN and 2-MN, respectively.

Example 1

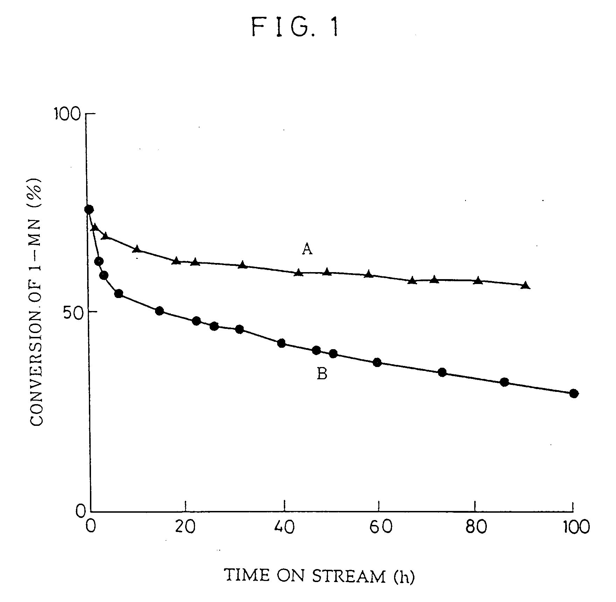

Y zeolite (0.5 g) that was dealuminated by steam treatment and that was protonically exchanged at cation sites (SiO$_2$/Al$_2$O$_3$ molar ratio = 18; lattice constant = 2.430 nm (24.30 Å)) was packed into a stainless steel reaction tube and reaction was performed at 450°C and under atmospheric pressure, with hydrogen and a feed being supplied at respective rates of 20 Nml/min and 0.75 g/h. The feed was 1-MN containing 10 wt% of tetralin and 0.07 wt%, in terms of nitrogen, of a nitrogen containing compound. The conversion of 1-MN was measured at predetermined time intervals and the results are plotted as curve A in Fig. 1. The conversion of 1-MN is expressed by [1 - (1-MN)/x] x 100 (%) where (1-MN) represents the molar fraction of 1-MN in the total product containing tetralin, and x represents the molar fraction of 1-MN in the total feed containing tetralin, and this parameter indicates how much of the 1-MN supplied is converted to substances other than 1-MN. The selectivity for 2-MN (the molar percent of the converted 1-MN that has become 2-MN) was low in the initial period of the reaction but thereafter it remained substantially at 90% or above.

Comparative Example 1

Reaction was performed in entirely the same manner as in example 1 except that the feed was solely composed of 1-MN. The conversion of 1-MN was measured at predetermined time intervals and the results are plotted as curve B in Fig. 1. The selectivity for 2-MN was 90%.

Example 2

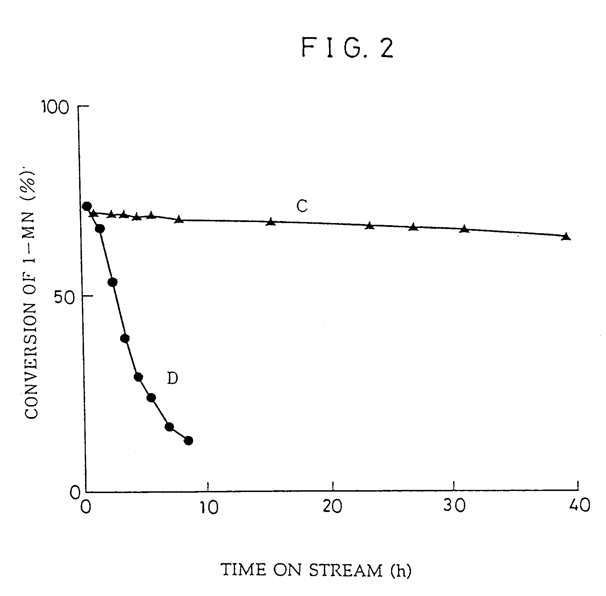

Reaction was performed in entirely the same manner as in Example 1 except that the catalyst was Y zeolite that was protonically exchanged at cation sites ($SiO_2/Al_2O_3$ molar ratio = 4.9; lattice constant = 2.463 nm (24.63 Å)). The conversion of 1-MN was measured at predetermined time intervals and the results are plotted as curve C in Fig. 2. The selectivity for 2-MN remained substantially at 90% or above.

Comparative Example 2

Reaction was performed in entirely the same manner as in Example 2 except that the feed was solely composed of 1-MN. The conversion of 1-MN was measured at predetermined time intervals and the results are plotted as curve D in Fig. 2. The selectivity for 2-MN was 90%.

Example 3

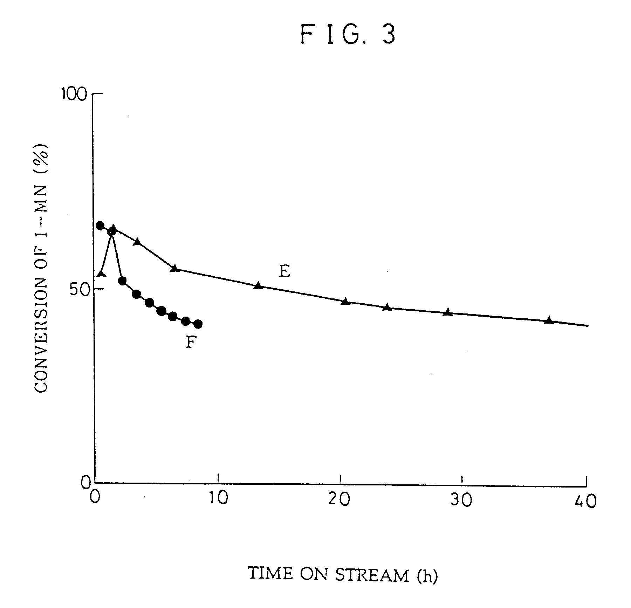

Reaction was performed in entirely the same manner as in Example 1 except that the catalyst was Silica Alumina 631 HN of Nikki Chemical Co., Ltd. The conversion of 1-MN was measured at predetermined time intervals and the results are plotted as curve E in Fig. 3. The selectivity for 2-MN remained at 95% or above.

Comparative Example 3

Reaction was performed in entirely the same manner as in Example 3 except that the feed was solely composed of 1-MN. The conversion of 1-MN was measured at predetermined time intervals and the results are plotted as curve F in Fig. 3. The selectivity for 2-MN was 95%.

Example 4

A feed (1-MN containing no nitrogen compounds) was subjected to reaction using the same catalyst and under the same reaction conditions as employed in Example 1. At 1450 h, the feed was changed to 1-MN containing 1 wt% methyltetralin and the reaction was continued. The results are shown in Table 1.

Example 5

Reaction was performed using the same catalyst and under the same reactions as employed in Example 4, except that hydrogen gas was supplied at a flow rate of 3 Nml/min whereas the feed was 1-MN containing 1 wt% methyltetralin. The conversion of 1-MN was 60%, the yield of 2-MN was 55% and the selectivity for 2-MN was 92 %. When the reaction was continued for 400 h under the same conditions, each of the parameters varied by less than 1%. The content of methyltetralin in the total product was 0.2 wt%.

Example 6

Reaction was performed using the same catalyst and under the same reaction conditions as employed in Example 4, except that hydrogen gas was supplied at a flow rate of 100 Nml/min whereas 1-MN containing 5 wt% methyltetralin was supplied as the feed in an amount of 3.75 g/h. The conversion of 1-MN was 66%, the yield of 2-MN was 64%, and the selectivity for 2-MN was 97%. When the reaction was continued for 100 h under the same conditions, each of the parameters varied by less than 1%. The content of methyltetralin in the total product was 3.0 wt%.

Example 7

The same catalyst as used in Example 4 was packed into a reaction tube as in Example 4 and reaction was performed at 430°C under atmospheric pressure with the feed being supplied in an amount of 0.75 g/h in the absence of any diluent gas. The feed was 1-MN containing 5% methyltetralin. The conversion of 1-MN was 58%, the yield of 2-MN was 51% and the selectivity for 2-MN was 88%. When the reaction was continued for 150 h under the same conditions, each of the parameters varied by less than 1%. The content of methyltetralin in the total product was 2.5 wt%.

Table 1

| Reaction time (h) | Conversion of 1-MN (%) | Yield of 2-MN (%) | Selectivity for 2-MN(%) |
|---|---|---|---|
| 10 | 68 | 60 | 88 |
| 100 | 60 | 53 | 88 |
| 1000 | 50 | 46 | 92 |
| 1450 (methyltetralin added) | 38 | 35 | 92 |
| 1500 | 48 | 46 | 95 |
| 1600 | 55 | 52 | 95 |
| 1800 | 63 | 60 | 95 |

Notes: 2-MN in thermodynamic

equilibrium = $2\text{-MN}/(1\text{-MN}) + (2\text{-MN}) \simeq 65\%$

$$\text{Conversion of 1-MN} = [1 - \frac{(1\text{-MN})}{X}] \times 100\%$$

$$\text{Yield of 2-MN} = \frac{(2\text{-MN})}{X} \times 100\%$$

$$\text{Selectivity for 2-MN} = \frac{\text{Yield}}{\text{Conversion}} \times 100 \ (\%)$$

where (1-MN): the molar fraction of 1-methylnaphthalene in the total product including methyltetralin

X : the molar fraction of 1-methylnaphthalene in the total feed including methyltetralin

(2-MN): the molar fraction of 2-methylnahthalene in the total product including methyltetralin

Example 8

The results of an experiment for 2-MN production that was conducted in accordance with the flowsheet of Fig. 4 are described specifically. The data for typical fractions obtained in the initial period of the operation are given in Table 2. Using a fixed-bed tubular flow reactor packed with a commercial hydrodesulfurizing catalyst (cobalt/molybdenum on alumina), a 1-MN containing oil 1 that was free from tar bases and indole (methylnaphthalene content = 94.9 wt%; concentration of sulfur compounds = 3.0 wt%) was hydrodesulfurized under the following conditions: temperature, 300°C; pressure, 2 kg/cm$^2$G; LHSV, 1.0 (L/L•hr); GHSV, 100 (L/L•hr), whereby the fraction 3 was freed of sulfur compounds to a level of 0.3 wt%. In the next distillation step (A), fraction 5 was distilled off in the distillation column 4 to obtain fraction 6 containing 2-MN in an amount of 64.7 wt%. Subsequently, the product 2-MN fraction 8 (product purity = 98.0 wt%; concentration of sulfur compounds = 0.3 wt%) was recovered from the distillation column 7. The recovery of 2-MN in fraction 8 was 80 wt% on the basis of 2-MN contained in fraction 1. Fraction 10 which was the combined flow of fractions 5 and 9 that had been distilled off in the distillation step (A) contained 2.6 wt% methyltetralin, of which 1-MN comprised 67.2 wt%. The proportion of methylnaphthalene in the fraction 10 that was occupied by 2-MN was 25 wt%.

In the next step of isomerization, the fraction 10 was passed through a catalyst bed packed with a solid acid catalyst (dealuminated Y zeolite protonically exchanged at cation sites; SiO$_2$/Al$_2$O$_3$ molar ratio = 18; lattice constant = 2.430 nm (24.30 Å)), and reaction was performed at a temperature of 450°C and at a WHSV (the weight of the feed passing over a unit weight of the catalyst per hour) of 1.5 h$^{-1}$, whereby 1-MN in the fraction 10 was isomerized to 2-MN until it comprised 62 wt% of the total methylnaphthalene. In the next distillation step (B), methylnaphthalene containing fraction 17 (2-MN content = 66.2 wt%) was recovered from the isomerized fraction 12. When the fraction 17 was circulated to combine with fraction 6, the recovery of 2-MN in fraction 8 increased up to 126 wt% on the basis of 2-MN in fraction 1. The activity of the isomerizing catalyst was maintained at satisfactory level throughout the operation for 330 days. That is, the content of 2-methylnaphthalene of the fraction 12 in the isomerization step was 56.5 wt% in the initial period of operation and 50.0 wt% after 330 days.

Thus, in accordance with the process of the present invention comprising the steps of hydrodesulfurization, distillation (A), isomerization and distillation (B), the recovery of 2-MN could be improved markedly while at the same time the concentration of sulfur compounds in the product was drastically reduced.

Table 2     Results of Example 8

(Unit: wt%)

| Fraction No. | 1[*1] | 3 | 6 | 8 | 10 | 12 | 17 |
|---|---|---|---|---|---|---|---|
| 2-MN | 65.2 | 64.2 | 64.7 | 98.0 | 22.2 | 56.5 | 66.2 |
| 1-MN | 29.7 | 29.2 | 33.1 | 1.5 | 67.2 | 34.3 | 32.8 |
| MT[*2] | 0 | 1.5 | 0.1 | 0.1 | 2.6 | 0.2 | 0.1 |
| Others | 2.1 | 4.8 | 1.8 | 0.1 | 7.7 | 8.7 | 0.6 |
| Sulfur compound | 3.0 | 0.3 | 0.3 | 0.3 | 0.3 | 0.3 | 0.3 |
| Recovery of 2-MN | | | | 126[*4] | | | |
| 2-MN/MN[*3] | 69 | 69 | 66 | 99 | 25 | 62 | 67 |

*1: Fraction 1 was the methylnaphthalene-containing feed oil.

*2: MT is the abbreviation for methyltetralin.

*3: MN is the sum of 2-MN and 1-MN.

*4: For the case where fraction 17 was circulated to

distillation column 7 (the recovery of 2-MN was

calculated on the basis of the amount of 2-MN in the 1-MN

containing oil (fraction 1).)

Comparative Example 4

The procedure of Example 8 was repeated except that fraction 5(methyltetralin-containing fraction) distilled off in the distillation step (A) was not supplied to the isomerizing step. The activity of the isomerizing step had a tendency to deteriorate gradually and after 80 days of the operation, 1-MN could no longer be isomerized to 2-MN.

Example 9

The results of an experiment for 2-MN production that was conducted in accordance with the flowsheet of Fig. 5 are described specifically. The data for typical fractions obtained in the initial period of the operation are given in Table 3.

As in Example 8, a 1-MN containing oil 101 that was free from tar bases and indole (sum content of 1-MN and 2-MN = 80 wt%; concentration of sulfur compounds = 2.8 wt%) was hydrodesulfurized, whereby the fraction 103 was freed of sulfur compounds to a level of 0.4 wt%. The fraction 103 contained tetralin and methyltetralin in a total amount of 1.0 wt% (tetralin/methyltetralin molar ratio = 1/9; degree of nuclear hydrogenation = 1.1 mol%) and 2-MN comprised 25 wt% of the methylnaphthalene.

In the next step of isomerization, the fraction 103 was passed through a catalyst bed packed with the same solid acid catalyst as used in Example 8 and an isomerization reaction was performed under the same conditions as those employed in Example 8. As a result, fraction 119 was obtained in which isomerization of 1-MN to 2-MN had proceeded until 2-MN comprised 60 wt% of the total methylnaphthalene.

The fraction 119 was subjected to distillation, in which the low-boiling oil was separated away in the distillation column 124 whereas the high-boiling oil was separated away in the distillation column 125, whereby the 2-MN fraction 122 was recovered as the final product.

The recovery of 2-MN in the product was 144 wt% on the basis of the fraction 101; the product purity was 97.0 wt%; and the concentration of sulfur compounds was 0.4 wt%.

The activity of the isomerizing catalyst was maintained at satisfactory level throughout the operation for 150 days; the content of 2-methylnaphthalene of the fraction 119 was 48.5 wt% in the initial period of the isomerizing operation and 45.0 wt% after 150 days.

### Table 3    Result of Example 9

(Unit: wt%)

| Fraction No. | 101 | 103 | 119 | 122 |
|---|---|---|---|---|
| 2-MN | 20 | 19.6 | 48.5 | 97.0 |
| 1-MN | 60 | 59.4 | 27.4 | 2.0 |
| Tetralins*1 | 0.0 | 1.0 | 0.1 | 0.1 |
| Others | 17.2 | 19.6 | 23.6 | 0.5 |
| Sulfur compound | 2.8 | 0.4 | 0.4 | 0.4 |
| Recovery of 2-MN* | | | | 144 |
| 2-MN/MN | | 25 | 64 | |

*1: Sum of tetralin and methyltetralin

*2: Recovery was calculated on the basis of the amount of 2-MN contained in fraction 101.

Example 10

Using Y zeolite protonically replaced at cation sites (lattice constant: 2.430 nm (24.30 Å)) as a catalyst, a reaction for isomerizing 1-MN to 2-MN was performed for 1000 h at 450°C and under atmospheric pressure with WHSV and $H_2$/(1-MN) volume ratio being adjusted to 1.5 h$^{-1}$ and 10, respectively. Thereafter, the feed was changed to 1-MN containing 1 wt% methyltetralin and the catalyst was regenerated as the isomerization reaction was performed for 600 h. Following this regeneration process, the feed was changed back to 1-MN and its isomerization was continued. The time-dependent profiles of the conversion of 1-MN and the yield of 2-MN in Example 10 are shown in Table 4.

Table 4

| Reaction time, h | Feed composition | Conversion of 1-MN, % | Yield of 2-MN, % |
|---|---|---|---|
| 100 | 1-MN100% | 70 | 62 |
| 500 | 1-MN100% | 64 | 60 |
| 1000 | 1-MN100% | 40 | 37 |
| 1100 | * | 62 | 60 |
| 1600 | * | 70 | 65 |
| 1700 | 1-MN100% | 61 | 58 |

*: 1-Methylnaphthalene containing 1 wt% methyltetralin

Example 11

As a hydrogenation catalyst, 1 g of nickel catalyst carried on a diatomaceous earth was packed into a stainless steel reactor and hydrogenation reaction was performed at 200 °C and under atmospheric pressure with hydrogen and 1-MN being supplied at respective rate of 10 Nml/min and 1 g/h. Together with the hydrogen, the resulting hydrogenated 1-MN liquid was passed through a stainless steel reaction tube packed with 1 g of Y zeolite which was protonically exchanged at cation sites ($SiO_2/Al_2O_3$ molar ratio = 25; lattice constant = 2.430 nm (24.30 Å)) and isomerized at 450 °C and under atmospheric pressure.

The conversion of 1-MN was 69.6 % in the initial period of the isomerization reaction. When reaction was continued for 100 h, the conversion of 1-MN was 62.5 %.

The selectivity for 2-MN was low in the initial period of the isomerization, however, it remained substantially at 90 % or above.

Methytetralin was found in the isomerized reaction mixture on an amount of 0.1 - 0.2 wt%.

Comparative Example 5

The same procedures of Example 11 were performed, except that the hydrogenation for 1-MN was not executed.

The conversion was same as the example 11 in the initial period of the isomerization, but after 100 hr it dropped to 21.7 %.

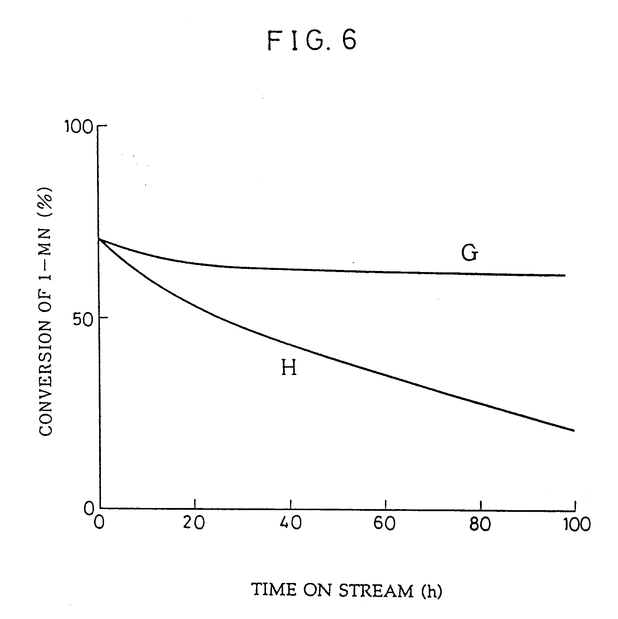

The conversions of 1-MN in the Example 11 and Comparative Example 5 are shown as curve G and H respectively in Fig. 6.

**Claims**

1. A process for producing 2-methylnaphthalene which comprises performing an isomerization reaction on a 1-methylnaphthalene containing feed oil by contacting said feed oil with a solid acid catalyst in the presence of at least one compound selected from the group consisting of tetralin and alkyltetralins, and recovering 2-methylnaphthalene from the resulting reaction product.

2. A process according to claim 1, wherein said solid acid catalyst is Y zeolite.

3. A process according to claim 2, wherein said solid acid catalyst is dealuminated Y zeolite.

4. A process according to claim 1, wherein at least one compound selected from the group consisting of tetralin and alkyltetralins is admixed to the feed oil before it is supplied to the reactor or fed to the reactor separately from the feed oil.

5. A process according to any one of claims 1 to 4, wherein the isomerization reaction is performed in the presence of methyltetralin.

6. A process according to any one of claims 1 to 5 wherein the content of nitrogen containing compound present as an impurity in the 1-methylnaphthalene containing feed oil is no more than 0.2 wt% as calculated for the nitrogen atom.

7. A process of claim 1, wherein 1-methylnaphthalene containing oil is subjected to a hydrodesulfurization step prior to the isomerization reaction step and part or all of the by-product methyltetralin of the hydrodesulfurization step is supplied to the isomerization step.

8. A process according to claim 7, wherein the total content of 1-methylnaphthalene and 2-methylnaphthalene in the 1-methylnaphthalene containing oil to be hydrodesulfurized is at least 80 wt%.

9. A process according to claim 7, wherein the content of methyltetralin in the feed oil to the isomerization step is in the range of 0.1 - 10.0 wt%.

10. A process according to claim 7, wherein the total content of tetralin and methyltetralin in the feed oil to the isomerization step is in the range of 0.1 - 10.0 wt%.

11. A process according to claim 7, wherein a catalyst carrying both molybdenum and at least one of cobalt and nickel is used in said hydrodesulfurization step.

12. A process according to any one of claims 7 to 11 that further comprises:
    before the isomerization step, subjecting the hydrodesulfurized oil to distillation step (A) together with the reaction product from the ensuing isomerizing step;
    fractionating the feed to the distillation step (A) into a methyltetralin fraction, a 2-methylnaphthalene fraction and a fraction consisting of 1-methylnaphthalene and 2-methylnaphthalene, with the 2-methylnaphthalene fraction being recovered as the product;
    supplying at least part of the methyltetralin fraction, as well as the fraction consisting of 1-methylnaphthalene and 2-methylnaphthalene to the isomerizing step;
    after the isomerization step, subjecting the reaction product from the isomerization step to distillation step (B) so that fractions having lower and higher boiling points than methylnaphthalene are rejected to the outside of the system; and
    circulating the remainder to the distillation step (A).

13. A process of claims 1 to 6 wherein the 1-methylnaphthalene containing feed oil is a hydrodesulfurized, methyltetralin containing 1-methylnaphthalene containing feed oil.

14. A process according to claim 13, wherein the total content of 1-methylnaphthalene and 2-methylnaphthalene in the 1-methylnaphthalene containing oil to be hydrodesulfurized is at least 80 wt%.

15. A process according to claim 13, wherein the content of methyltetralin in the feed oil to the isomerizing step is in the range of 0.1 - 10.0 wt%.

16. A process according to claim 13, wherein the total content of tetralin and methyltetralin in the feed oil to the isomerizing step is in the range of 0.1 - 10.0 wt%.

17. A process according to claim 13, wherein a catalyst carrying both molybdenum and at least one of cobalt and nickel is used in said hydrodesulfurization step.

18. A process according to claim 13, wherein 2-methylnaphthalene is recovered by distillation.

19. A process of claim 1, wherein 1-methylnaphthalene containing oil is subjected to a hydrogenation step prior to the isomerization reaction step and part or all of the by-product methyltetralin of the hydrogenation step is supplied to the isomerization step.

20. A process according to claim 19, wherein the total content of 1-methylnaphthalene and 2-methylnaphthalene in the 1-methylnaphthalene containing oil to be hydrogenated is at least 80 wt%.

17

EP 0 490 349 B1

21. A process according to claim 19, wherein a catalyst carrying both molybdenum and at least one of cobalt and nickel is used in said hydrogenation step.

22. A process of claims 1 to 6 wherein the 1-methylnaphthalene containing feed oil is a hydrogenated, methyltetralin containing 1-methylnaphthalene containing feed oil.

23. A process according to claim 22, wherein the total content of 1-methylnaphthalene and 2-methylnaphthalene in the 1-methylnaphthalene containing oil to be hydrogenated is at least 80 wt%.

24. A process according to claim 22, wherein a catalyst carrying both molybdenum and at least one of cobalt and nickel is used in said hydrogenation step.

25. A method of restoring the activity of a solid acid catalyst for isomerization of 1-methylnaphthalene to 2-methylnaphthalene, in which a solid acid catalyst whose activity has deteriorated as a result of its use in the reaction of isomerization of a 1-methylnaphthalene containing feed oil is brought into contact under reaction conditions for isomerization with at least one compound selected from the group consisting of tetralin and alkyltetralins.

26. A method according to claim 25, wherein said reaction conditions for isomerization are such that the temperature is 300 - 600 °C, the pressure is 0 - 10 kg/cm$^2$G, WHSV is 0.1 - 20 h$^{-1}$, said compound comprises at least 0.01 wt% of the sum of said feed oil and said compound, and that the volume ratio of a diluent gas to the gasified feed oil is 0 - 100.

27. A method according to claim 25, wherein the solid acid catalyst is Y zeolite.

28. A method according to claim 27, wherein the solid acid catalyst is dealuminated Y zeolite.

29. A method according to claim 25, wherein the deteriorated solid acid catalyst is brought into contact with methyltetralin.

**Patentansprüche**

1. Verfahren zur Herstellung von 2-Methylnaphthalin durch Durchführen einer Isomerisierungsreaktion bei einem 1-Methylnaphthalin enthaltenden Beschickungsöl, indem das Beschickungsöl mit einem festen, sauren Katalysator in Gegenwart mindestens einer unter Tetralin und Alkyltetralinen ausgewählten Verbindung in Berührung gebracht wird, und Gewinnen von 2-Methylnaphthalin aus dem erhaltenen Reaktionsprodukt.

2. Verfahren nach Anspruch 1, wobei der feste, saure Katalysator ein Y-Zeolith ist.

3. Verfahren nach Anspruch 2, wobei der feste, saure Katalysator ein dealuminierter Y-Zeolith ist.

4. Verfahren nach Anspruch 1, wobei mindestens eine unter Tetralin und Alkyltetralinen ausgewählte Verbindung mit dem Beschickungsöl vor Einbringen desselben in den Reaktor vermischt oder dem Reaktor getrennt von dem Beschickungsöl zugespeist wird.

5. Verfahren nach einem der Ansprüche 1 bis 4, wobei die Isomerisierungsreaktion in Gegenwart von Methyltetralin erfolgt.

6. Verfahren nach einem der Ansprüche 1 bis 5, wobei der Gehalt einer stickstoffhaltigen Verbindung, die in dem 1-Methylnaphthalin enthaltenden Beschickungsöl als Verunreinigung vorliegt, nicht mehr als 0,2 Gew.-%, berechnet für das Stickstoffatom, beträgt.

7. Verfahren nach Anspruch 1, wobei das 1-Methylnaphthalin enthaltende Öl vor der Isomerisierungsreaktion einer Hydrodesulfurierungsstufe unterzogen und ein Teil des oder das gesamte Nebenprodukt(s) Methyltetralin aus der Hydrodesulfurierungsstufe der Isomerisierungsstufe zugespeist wird.

8. Verfahren nach Anspruch 7, wobei der Gesamtgehalt an 1-Methylnaphthalin und 2-Methylnaphthalin in dem einer Hydrodesulfurierung zu unterziehenden, 1-Methylnaphthalin enthaltenden Öl mindestens 80 Gew.-% beträgt.

9. Verfahren nach Anspruch 7, wobei der Methyltetralingehalt im Beschickungsöl für die Isomerisierungsstufe in einem Bereich von 0,1 bis 10,0 Gew.-% liegt.

10. Verfahren nach Anspruch 7, wobei der Gesamtgehalt an Tetralin und Methyltetralin im Beschickungsöl für die Isomerisierungsstufe in einem Bereich von 0,1 bis 10,0 Gew.-% liegt.

11. Verfahren nach Anspruch 7, wobei in der Hydrodesulfurierungsstufe ein Katalysator verwendet wird, der sowohl Molybdän als auch Kobalt und/oder Nickel trägt.

12. Verfahren nach einem der Ansprüche 7 bis 11, des weiteren umfassend:
vor der Isomerisierungsstufe eine Destillation (A) des hydrodesulfurierten Öls zusammen mit dem Reaktionsprodukt aus der nachfolgenden Isomerisierungsstufe;
ein Fraktionieren der Beschickung für die Destillationsstufe (A) in eine Methyltetralinfraktion, eine 2-Methylnaphthalinfraktion und eine aus 1-Methylnaphthalin und 2-Methylnaphthalin bestehende Fraktion, wobei die 2-Methylnaphthalinfraktion als Produkt gewonnen wird,
Einspeisen mindestens eines Teils der Methyltetralinfraktion sowie der aus 1-Methylnaphthalin und 2-Methylnaphthalin bestehenden Fraktion in die Isomerisierungsstufe;
nach der Isomerisierungsstufe Unterwerfen des Reaktionsproduktes aus der Isomerisierungsstufe einer Destillationsstufe (B), so daß Fraktionen mit niedrigeren und höheren Siedepunkten als Methylnaphthalin aus dem System ausgetragen werden, und
Zurückzirkulierenlassen des Rests in die Destillationsstufe (A).

13. Verfahren nach einem der Ansprüche 1 bis 6, wobei das 1-Methylnaphthalin enthaltende Beschickungsöl ein hydrodesulfuriertes, Methyltetralin enthaltendes, 1-Methylnaphthalin enthaltendes Beschickungsöl ist.

14. Verfahren nach Anspruch 13, wobei der Gesamtgehalt an 1-Methylnaphthalin und 2-Methylnaphthalin in dem 1-Methylnaphthalin enthaltenen Öl, das einer Hydrodesulfurierung zu unterziehen ist, mindestens 80 Gew.-% beträgt.

15. Verfahren nach Anspruch 13, wobei der Gehalt an Methyltetralin in dem Beschickungsöl für die Isomerisierungsstufe in einem Bereich von 0,1 bis 10,0 Gew.-% liegt.

16. Verfahren nach Anspruch 13, wobei der Gesamtgehalt an Tetralin und Methyltetralin im Beschickungsöl für die Isomerisierungsstufe im Bereich von 0,1 bis 10,0 Gew.-% liegt.

17. Verfahren nach Anspruch 13, wobei in der Hydrodesulfurierungsstufe ein Katalysator verwendet wird, der sowohl Molybdän als auch Kobalt und/oder Nickel trägt.

18. Verfahren nach Anspruch 13, wobei 2-Methylnaphthalin durch Destillation gewonnen wird.

19. Verfahren nach Anspruch 1, wobei ein 1-Methylnaphthalin enthaltendes Öl vor der Isomerisierungsreaktion einer Hydrierungsstufe unterzogen wird und ein Teil des oder das gesamte Nebenprodukt(s) Methyltetralin der Hydrierungsstufe der Isomerisierungsstufe zugespeist wird.

20. Verfahren nach Anspruch 19, wobei der Gesamtgehalt an 1-Methylnaphthalin und 2-Methylnaphthalin in dem zu hydrierenden, 1-Methylnaphthalin enthaltenden Öl mindestens 80 Gew.-% beträgt.

21. Verfahren nach Anspruch 19, wobei in der Hydrierungsstufe ein Katalysator verwendet wird, der sowohl Molybdän als auch Kobalt und/oder Nickel trägt.

22. Verfahren nach einem der Ansprüche 1 bis 6, wobei das 1-Methylnaphthalin enthaltende Beschickungsöl ein hydriertes, Methyltetralin enthaltendes, 1-Methylnaphthalin enthaltendes Beschickungsöl ist.

**23.** Verfahren nach Anspruch 22, wobei der Gesamtgehalt an 1-Methylnaphthalin und 2-Methylnaphthalin in dem zu hydrierenden, 1-Methylnaphthalin enthaltenden Öl mindestens 80 Gew.-% beträgt.

**24.** Verfahren nach Anspruch 22, wobei in der Hydrierungsstufe ein Katalysator verwendet wird, der sowohl Molybdän als auch Kobalt und/oder Nickel trägt.

**25.** Verfahren zur Wiederherstellung der Aktivität eines festen, sauren Katalysators zur Isomerisierung von 1-Methylnaphthalin in 2-Methylnaphthalin, bei dem ein fester, saurer Katalysator, dessen Aktivität als Ergebnis seiner Verwendung bei der Isomerisierungsreaktion eines 1-Methylnaphthalin enthaltenden Beschickungsöls verschlechtert worden ist, unter Reaktionsbedingungen für eine Isomerisierung mit mindestens einer unter Tetralin und Alkyltetralinen ausgewählten Verbindung in Berührung gebracht wird.

**26.** Verfahren nach Anspruch 25, wobei die Reaktionsbedingungen für eine Isomerisierung derart sind, daß die Temperatur 300 bis 600°C, der Druck 0 bis 10 kg/cm$^2$ (Meßdruck) und WHSV 0,1 bis 20 h$^{-1}$ betragen, die Verbindung mindestens 0,01 Gew.-% der Summe aus dem Beschickungsöl und der Verbindung umfaßt und das Volumenverhältnis Verdünnungsgas/in einen gasförmigen Zustand überführtes Beschickungsöl 0 bis 100 beträgt.

**27.** Verfahren nach Anspruch 25, wobei der feste, saure Katalysator ein Y-Zeolith ist.

**28.** Verfahren nach Anspruch 27, wobei der feste saure Katalysator ein dealuminierter Y-Zeolith ist.

**29.** Verfahren nach Anspruch 25, wobei der verschlechterte feste, saure Katalysator mit Methyltetralin in Berührung gebracht wird.

**Revendications**

**1.** Un procédé pour la production de 2-méthylnaphtalène, qui comporte l'exécution d'une réaction d'isomérisation sur une huile d'alimentation contenant du 1-méthylnaphtalène en mettant en contact ladite huile d'alimentation avec un catalyseur acide solide en présence d'au moins un composé sélectionné dans le groupe constitué par la tétraline et les alkyltétralines, et en récupérant le 2-méthylnaphtalène du produit de réaction résultant.

**2.** Un procédé selon la revendication 1, dans lequel ledit catalyseur acide solide est de la zéolite Y.

**3.** Un procédé selon la revendication 2, dans lequel ledit catalyseur acide solide est de la zéolite Y désaluminée.

**4.** Un procédé selon la revendication 1, dans lequel au moins un composé sélectionné dans le groupe constitué par la tétraline et les alkyltétralines est ajouté en mélange à l'huile d'alimentation avant qu'elle soit délivrée au réacteur ou délivrée au réacteur séparément de l'huile d'alimentation.

**5.** Un procédé selon l'une quelconque des revendications 1 à 4, dans lequel la réaction d'isomérisation est exécutée en présence de méthyltétraline.

**6.** Un procédé selon l'une quelconque des revendications 1 à 5, dans lequel la teneur en composé contenant de l'azote et présent comme impureté dans l'huile d'alimentation contenant du 1-méthylnaphtalène n'est pas supérieure à 0,2% en poids, le calcul étant fait pour l'atome d'azote.

**7.** Un procédé selon la revendication 1, dans lequel l'huile contenant du 1-méthylnaphtalène est soumise à une phase d'hydrodésulfuration avant la phase de réaction d'isomérisation, et tout ou partie du sous-produit méthyltétraline de la phase d'hydrodésulfuration est délivré à la phase d'isomérisation.

**8.** Un procédé selon la revendication 7, dans lequel la teneur totale en 1-méthylnaphtalène et 2-méthylnaphtalène dans l'huile contenant du 1-méthylnaphtalène à hydrodésulfurer est d'au moins 80 % en poids.

9. Un procédé selon la revendication 7, dans lequel la teneur en méthyltétraline dans l'huile d'alimentation délivrée à la phase d'isomérisation est située dans la plage de 0,1 - 10,0 % en poids.

10. Un procédé selon la revendication 7, dans lequel la teneur totale de tétraline et de méthyltétraline dans l'huile d'alimentation délivrée à la phase d'isomérisation est située dans la plage de 0,1 - 10,0 % en poids.

11. Un procédé selon la revendication 7, dans lequel un catalyseur portant à la fois du molybdène et au moins l'un du cobalt et du nickel est utilisé dans ladite phase d'hydrodésulfuration.

12. Un procédé selon l'une quelconque des revendications 7 à 11, qui comprend au surplus :
   avant la phase d'isomérisation, la soumission de l'huile hydrodésulfurée à une phase (A) de distillation conjointement avec le produit de réaction provenant de la phase d'isomérisation qui s'ensuit ;
   le fractionnement de l'alimentation à la phase de distillation (A) en une fraction méthyltétraline, une fraction 2-méthylnaphtalène et une fraction constituée de 1-méthylnaphtalène et 2-méthylnaphtalène, la fraction 2-méthylnaphtalène étant récupérée en tant que produit ;
   la fourniture d'au moins une partie de la fraction méthyltétraline, de même que de la fraction constituée de 1-méthylnaphtalène et 2- méthylnaphtalène , à la phase d'isomérisation ;
   avant la phase d'isomérisation , la soumission du produit de réaction provenant de la phase d'isomérisation à une phase de distillation (B) de manière que les fractions présentant des points d'ébullition inférieurs et supérieurs au méthylnaphtalène soient rejetées à l'extérieur du système; et
   la circulation du reste vers la phase de distillation (A).

13. Un procédé des revendications 1 à 6, dans lequel l'huile d'alimentation contenant du 1-méthylnaphtalène est une huile d'alimentation hydrodésulfurée contenant du 1-méthylnaphtalène contenant de la méthyltétraline.

14. Un procédé selon la revendication 13, dans lequel la teneur totale en 1-méthylnaphtalène et 2-méthylnaphtalène dans l'huile contenant du 1-méthylnaphthalène à hydrodésulfurer est d'au moins 80 % en poids.

15. Un procédé selon la revendication 13, dans lequel la teneur de méthyltétraline dans l'huile d'alimentation délivrée à la phase d'isomérisation est située dans la plage de 0,1 - 10,0 % en poids.

16. Un procédé selon la revendication 13, dans lequel la teneur totale de tétraline et méthyltétraline dans l'huile d'alimentation délivrée à la phase d'isomérisation est située dans la plage de 0,1 - 10,0 % en poids.

17. Un procédé selon la revendication 13, dans lequel un catalyseur portant à la fois du molybdène et au moins l'un du cobalt et du nickel est utilisé dans ladite phase d'hydrodésulfuration.

18. Un procédé selon la revendication 13, dans lequel le 2-méthylnaphtalène est récupéré par distillation.

19. Un procédé selon la revendication 1, dans lequel l'huile contenant du 1-méthylnaphtalène est soumise à une phase d'hydrogénation avant la phase de réaction d'isomérisation, et tout ou partie du sous-produit méthyltétraline de la phase d'hydrogénation est délivré à la phase d'isomirésation.

20. Un procédé selon la revendication 19, dans lequel la teneur totale de 1-méthylnaphtalène et 2-méthylnaphtalène dans l'huile contenant du 1-méthylnaphtàlène à hydrogéner est d'au moins 80 % en poids.

21. Un procédé selon la revendication 19, dans lequel un catalyseur portant à la fois du molybdène et au moins l'un du cobalt et du nickel est utilisé dans ladite phase d'hydrogénation.

22. Un procédé selon les revendications 1 à 6, dans lequel l'huile d'alimentation contenant du 1-méthylnaphtalène est une huile d'alimentation hydrogénée contenant du 1-méthylnaphtalène contenant de la méthyltétraline.

EP 0 490 349 B1

**23.** Un procédé selon la revendication 22, dans lequel la teneur totale de 1-méthylnaphtalène et 2-méthylnaphtalène dans l'huile contenant du 1-méthylnaphtalène à hydrogéner est d'au moins 80 % en poids.

**24.** Un procédé selon la revendication 22, dans lequel un catalyseur portant à la fois du molybdène et au moins l'un du cobalt et du nickel est utilisé dans ladite phase d'hydrogénation.

**25.** Un procédé pour restaurer l'activité d'un catalyseur acide solide pour l'isomérisation de 1-méthylnaphtalène en 2-méthylnaphtalène , dans lequel un catalyseur acide solide, dont l'activité s'est détériorée du fait de son utilisation dans la réaction d'isomérisation d'une huile d'alimentation contenant du 1-méthylnaphtalène , est amené en contact, dans des conditions de réaction pour l'isomérisation, avec au moins un composé sélectionné dans le groupe constitué par la tétraline et les alkyltétralines.

**26.** Un procédé selon la revendication 25, dans lequel lesdites conditions de réaction pour l'isomérisation sont telles que la température est de 300-600°C, la pression est 0-10 kg/cm$^2$G, WHSV (poids de l'huile d'alimentation passant sur un poids unitaire de catalyseur par heure) est 0,1 - 20 h$^{-1}$, ledit composé comporte au moins 0,01 % en poids de la somme de ladite huile d'alimentation et dudit composé, et le rapport volumique d'un gaz diluant à l'huile d'alimentation gazéifiée est 0-100.

**27.** Un procédé selon la revendication 25, dans lequel le catalyseur acide solide est de la zéolite Y.

**28.** Un procédé selon la revendication 27, dans lequel le catalyseur acide solide est de la zéolite Y désaluminée.

**29.** Un procédé selon la revendication 25, dans lequel le catalyseur acide solide détérioré est amené en contact avec la méthyltétraline.

22

# F I G. 1

FIG. 2

# F I G. 3

TIME ON STREAM (h)

FIG. 4

DISTILLATION STEP (A)

8 PRODUCT

HYDRODESULFURI-ZATION

ISOMERIZATION

DISTILLATION STEP (B)

EP 0 490 349 B1

FIG. 5

FIG. 6